Europäisches Patentamt

**European Patent Office**

**Office européen des brevets**

(11) Numéro de publication: **0 186 542 B1**

# FASCICULE DE BREVET EUROPEEN

(45) Date de publication de fascicule du brevet: **05.08.92**

(51) Int. Cl.⁵: **A61K 47/00**, A61K 9/50, A61K 31/685

(21) Numéro de dépôt: **85402254.8**

(22) Date de dépôt: **20.11.85**

Le dossier contient des informations techniques présentées postérieurement au dépôt de la demande et ne figurant pas dans le présent fascicule.

(54) **Procédé de solubilisation, au moment de l'emploi, d'un principe actif lipophile dans une solution aqueuse, en vue de son administration par voie intraveineuse.**

(30) Priorité: **22.11.84 FR 8417792**

(43) Date de publication de la demande:
**02.07.86 Bulletin 86/27**

(45) Mention de la délivrance du brevet:
**05.08.92 Bulletin 92/32**

(84) Etats contractants désignés:
**CH DE GB IT LI**

(56) Documents cités:
**EP-A- 100 459**
**EP-A- 132 821**
**EP-A- 133 258**

**CHEMICAL ABSTRACTS, vol. 91, no. 22, 26 novembre 1979, page 387, no. 181378b, Columbus, Ohio, US; J.P. POLIDORO et al.: "Development of orally-active dosage forms for steroids", & REPORT 1979, NICHD/CPR/CDB-79/2; Order No. PB-292493, 178pp., AVAIL. NTIS. FROM GOV. REP. ANNOUNCE. INDEX (US) 1979, 79(12), 75**

(73) Titulaire: **Laboratoires BESINS ISCOVESCO Société anonyme dite :**
**5, rue du Bourg l'Abbé**
**F-75003 Paris(FR)**

(72) Inventeur: **Puisieux, Francis**
**66, rue de Strasbourg**
**F-94700 Maisons Alfort(FR)**
Inventeur: **Salin-Drouin, Dominique**
**Résidence la Baleine 4, rue du Mérou**
**F-92290 Chatenay Malabry(FR)**

(74) Mandataire: **Bruder, Michel**
**10 rue de la Pépinière**
**F-75008 Paris(FR)**

CHEMICAL ABSTRACTS, vol. 88, no. 26, 26 juin 1978, page 395, no. 197514s, Columbus, Ohio, US; G.P. MARTIN et al.: "The solubilization and dissolution of progresterone by bile salt mixtures", & J. PHARM. PHARMACOL. 1977, 29(Suppl., Br. Pharm. Conf. 1977) 20P

NATURE, vol. 192, no. 4800, 28 octobre 1961, pages 329-331, McMillan & Co., Ltd., Londres, GB; A.I. CSAPO: "Effect of progresterone on pregnancy"

**Description**

La présente invention concerne un procédé de solubilisation, AU moment de l'emploi, d'un principe actif lipophile dans une solution aqueuse, en vue de son administration par voie intraveineuse.

L'administration, par voie intraveineuse, d'un principe actif lipophile a déjà été résolue de différentes façons. L'un des procédés connus consiste à utiliser un mélange de solvants miscibles à l'eau mais ce procédé entraîne un risque de recristallisation si le volume aqueux devient trop important, par exemple après dilution dans une perfusion de NaCl à 9°/oo. On a également utilisé des agents hydrotropes comme le benzoate de sodium, ces agents n'étant toutefois utilisables que pour certains types de molécules. On a également employé la formation d'agrégats, par exemple dans le cas de la solubilisation de la progestérone par de la sérum albumine, mais à des doses telles que l'on rencontre des problèmes de toxicité.

Un autre procédé connu consiste en une injection dans du plasma préalablement prélevé chez le sujet à traiter, ainsi qu'il est décrit par EVERETT RB et coll. Am.J. obstet. Gynecol. (1978) 131, 352.

Un a également envisagé d'utiliser une solubilisation en milieu aqueux à l'aide de lécithine et d'un formateur de micelles, ainsi qu'il est décrit dans le brevet belge 856 675 et dans le brevet européen 0 011 745.

La présente invention vise à remédier aux inconvénients des procédés précités connus antérieurement.

A cet effet ce procédé de solubilisation, au moment de l'emploi, d'un principe actif lipophile dans une solution aqueuse, en vue de son administration par voie intraveineuse, est caractérisé en ce que l'on prépare une solution contenant au moins un principe actif lipophile, de la lécithine ou un autre phospholipide dont la température de transition de phase est inférieure à la température ambiante, au moins un agent conservateur et au moins un solvant organique (ou un mélange solvant-eau) atoxique par voie intraveineuse, miscible à l'eau, dans lequel les autres composés sont solubles.

La solution ainsi préparée peut être introduite dans une perfusion de chlorure de sodium ou de glucose sans qu'il y ait relargage du principe actif pendant au moins 48 heures, à condition que la quantité de lécithine soit suffisante.

Le procédé suivant l'invention offre plusieurs avantages. En premier lieu il permet d'utiliser un phospholipide, tel que la lécithine par exemple, dénué de toxicité par voie intraveineuse. Par ailleurs la quantité de solvant organique utilisée est très faible. Enfin les problèmes de stabilité sont limités par rapport à une solution initiale aqueuse de lécithine.

La technique qui consiste à injecter une solution alcoolique de lécithine dans de l'eau a été décrite par S. BATZRI et E.D. KORN "Single bilayer liposomes prepared without sonication", Biochem. Biophys. Act. (1973), 293, 1015-1019, pour obtenir des liposomes pouvant véhiculer un principe actif, cette injection étant suivie d'un chauffage ou d'une dialyse pour éliminer l'alcool.

On donnera ci-dessous, à titre illustratif, des exemples de préparation de solutions par le procédé suivant l'invention:

| Exemple 1 | |
|---|---|
| - Dihydrotestostérone | 2,5 mg |
| - Phosphatidylcholine d'oeuf (ovothin-lécithos) | 100 mg |
| - Palmitate d'ascorbyle | 0,4 mg |
| - Tampon phosphate pH 7,2 | 0,375 ml |
| - Alcool absolu    qsp | 2,5 ml |

| Exemple 2 | |
|---|---|
| - Progestérone | 10 mg |
| - Phosphatidylcholine de soja (Epikuron 200-lécithos) | 175 mg |
| - Palmitate d'ascorbyle | 0,6 mg |
| - Alcool absolu    qsp | 2,5 ml |

L'injection des solutions suivant les exemples 1 et 2 dans un flacon de perfusion de chlorure de sodium isotonique donne une légère opalescence due à la présence de vésicules dont la taille moyenne est d'une centaine de nanomètres. Par ailleurs l'observation au microscope électronique à transmission montre la formation de structures lamellaires closes ressemblant à des liposomes.

Au cours de tests médicaux on a injecté une solution de dihydrotestostérone (D.H.T.), obtenue suivant l'exemple 1, par voie intraveineuse, après dilution dans un soluté de perfusion de chlorure de sodium. Un premier patient a reçu 88,2 microgrammes par heure de D.H.T. pendant 4 heures, puis 176,4 microgrammes par heure de D.H.T. les 4 dernières heures restantes. Un second patient a reçu, durant ces mêmes périodes de temps, d'abord 176,4 microgrammes par heure puis 264,6 microgrammes par heure.

Les résultats des taux plasmatiques obtenus sont indiqués dans le diagramme annexé, les courbes 1 et 2 correspondant respectivement aux premier et second patients précités. Sur ce diagramme le temps t est indiqué en abcisses, en minutes, tandis que le taux plasmatique TP est porté en ordonnées.

On voit d'après ce diagramme que quelle que soit la dose injectée, la montée en plateau est obtenue rapidement et paraît stable. Il est possible d'atteindre des taux plasmatiques compatibles avec une utilisation thérapeutique. Par ailleurs les soltions sont très bien tolérées par les patients et n'entraînent aucun trouble d'intolérance.

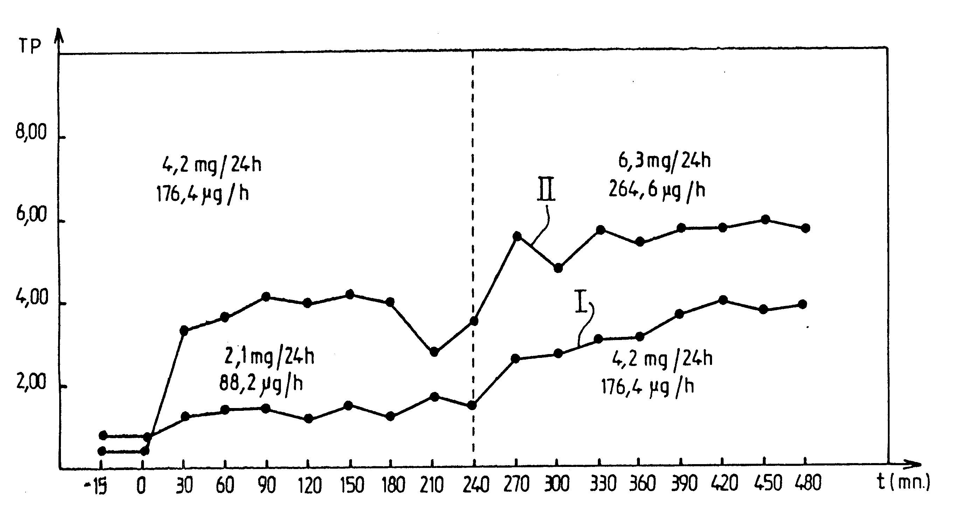

**Revendications**

**1.** Procédé de solubilisation au moment de l'emploi d'un principe actif lipophile dans du sérum physiologique pour administration intraveineuse caractérisé par le fait
- que l'on introduit au moins un principe actif lipophile, de la lécithine ou des phospholipides à température de transition de phase inférieure à la température ambiante dans au moins un solvant organique ou un mélange solvant organique/eau, atoxique par voie intraveineuse, les quantités des composants étant suffisantes pour solubiliser ledit principe actif,
- et que l'on mélange au moment de l'emploi la phase ainsi obtenue dans une quantité supérieure de phase aqueuse constituée par du sérum pour perfusion.

**2.** Procédé selon la revendication 1 caractérisé en ce que la solution contient un agent stabilisant.

**3.** Procédé selon l'une quelconque des revendications 1 ou 2 caractérisé en ce que le mélange obtenu à administrer par voie intraveineuse se présente sous forme d'une suspension liposomale.

**Claims**

**1.** Process for solubilizing, at the moment of use, a lipophilic active ingredient, in an physiological saline solution, for administration thereof by intravenous route, characterized in that :
- at least one lipophilic active ingredient, lecithin or phospholipids, are introduced, at a phase transition temperature lower than ambient temperature, in at least one organic solvent or a solvent/water mixture, non-toxic by intravenous route, the compounds quantities being sufficient for solubilizing said active ingredient,
- and, at the moment of use, the phase thus obtained is mixed in a higher quantity of an aqueous phase constituted by a serum or saline solution for perfusion.

**2.** Process according to claim 1, characterized in that said solution comprises a stabilizing agent.

**3.** Process according to any of the preceeding claims 1 or 2, characterized in that the obtained mixture for intravenous administration is a liposomal suspension.

**Patentansprüche**

**1.** Verfahren zur im Augenblick der Anwendung erfolgenden Lösung eines aktiven lipophilen Stoffes in einem physiologischen Serum für intravenöse Verabreichung, dadurch gekennzeichnet, dass man wenigstens einen aktiven lipophilen Stoff, Lecithin oder Phospholipide, mit einer unter der Umgabungstemperatur liegenden Phasenübergangstemperatur in wenigstens ein bei intravenöser Verabreichung ungiftiges organisches Lösungsmittel oder eine Mischung eines organischen Lösungsmittels mit Wasser einbringt, wobei die Mengen der Bestandteile zur Lösung des genannten aktiven Stoffes ausreichend sind,
- und dass man im Augenblick der Anwendung die so erhaltene Phase in eine grössere Menge der durch das Serum für die Perfusion gebildeten Phase mischt.

**2.** Verfahren nach Anspruch 1, dadurch gekennzeichnet, dass die Lösung einen Stabillsator enthält.

3. Verfahren nach einem der Ansprüche 1 oder 2, dadurch gekennzeichnet, dass zur intravenösen Verabreichung erhaltene Mischung die Form einer liposomalen Suspension aufweist.